# EUROPEAN PATENT APPLICATION

(11) **EP 3 893 091 A1**
(43) Date of publication of application: **13.10.2021**
(21) Application number: 20169062.5
(22) Date of filing: 09.04.2020
(51) Int. Cl.: G06F 3/01, G06F 3/0484, G16H 10/60, G16H 30/40, G06T 11/20

(54) **CONTROLLING A DATA INSPECTION BY A USER**

(71) Applicant: Koninklijke Philips N.V., 5656 AG Eindhoven (NL)
(72) Inventor: TALGORN, Elise Claude Valentine, 5656 AE Eindhoven (NL); LAUTE, Niels, 5656 AE Eindhoven (NL); BUIL, Vincentius Paulus, 5656 AE Eindhoven (NL)
(74) Representative: Philips Intellectual Property & Standards

(57) **Abstract**

Some embodiments are directed to a system 101 for controlling a data inspection by a user. A first visualization of the data is shown based on a first data representation setting and a first data content setting. A gaze pattern and cognitive state of the user are determined, based on which a second data content setting and a second data representation setting are determined. A second visualization of the data is shown based on the second data representation setting and the second data content setting.

## Description

### FIELD OF THE INVENTION

The invention relates to a system for controlling a data inspection by a user, a data inspection device for use in such a system, a method of controlling a data inspection by a user, and a computer-readable storage medium.

### BACKGROUND OF THE INVENTION

In the medical context, clinicians are being confronted by increasing amounts of increasingly complex data. For example, in tumor board meetings, clinicians may be presented with patient information of different kinds from multiple modalities, e.g., images such as scans, electronic medical records, comparisons with similar patients, etcetera. Based on this information, the clinicians have to make a decision, e.g., a diagnosis or a course of treatment, under high pressure, e.g., in a matter of minutes. Similarly, while performing a medical procedure, clinicians may have access to various real-time imaging and vital sign data, patient records, etcetera.

Various techniques are available to control a visualization of medical data to a user. For example, European patent EP0616290B1 proposes a medical information processing system for supporting diagnosis. A first medical image is shown. If an abnormality is detected in the first medical image, the attention of the user is drawn to the abnormality by showing a second medical image overlaid on the first medical image along with a marker indicating the location of the abnormality in the first medical image.

### SUMMARY OF THE INVENTION

For various reasons, a situation may arise wherein the user does not understand the data presented to him. For example, the user may not understand what data is presented to him and/or how that data is represented. If the user does not understand presented data, the user may lose time and may even quit the visualization without having fully understood the data, e.g., potentially misinterpreting or even missing critical information.

There is a need to automatically control a data inspection by a user to avoid such misunderstandings, in particular, in settings where it is hard for the user to make a manual selection of how data should be represented. For example, this can be the case if new data is presented to the user in quick succession such as in a tumor board meeting and/or if the user performs the data inspection as part of another process, e.g., a medical procedure. In such cases, selecting an appropriate representation of data, e.g., a representation that matches the cognitive needs of the user, in a substantially automatic way is desirable.

These and other problems of existing systems for controlling data inspection by a user are addressed by the system for controlling data inspection, data inspection device, and method of controlling a data inspection as defined by the claims.

The inventors realized that there are different ways in which a data inspection by a user can be controlled to make it better match the cognitive needs of the user. These ways relate to different reasons why a visualization shown to a user can be non-optimal. For example, in some cases, a graphical representation of the data may be non-optimal, so that a user may have difficulty or even be unable to understand the data. In other cases, the graphical representation may be appropriate but the content of the data is unclear to the user, so that a user would benefit from additional data to understand and interpret the data that is presented.

In the system as defined by the claims, a first visualization of the data may be shown to the user. This first visualization may be defined by a first data content setting, defining a selection of data to show, and a first data representation setting, defining how to show the selection of the data. Interestingly, feedback on how the user perceived the first visualization may be obtained using a gaze pattern sensor for sensing data indicative of a gaze pattern of the user and/or a cognitive state sensor for sensing data indicative of a cognitive state of the user. Based on the gaze pattern and the cognitive state, a second data content setting and a second data representation setting may be determined, according to which the data may be visualized and shown to the user.

Hence, for example, the system may detect that the user is confused when looking at the data because of the graphical presentation of the data, and adjust the data representation setting accordingly. Or, the system may detect that the user is confused because of the content of the data, and adjust the data content setting accordingly. In any case, misunderstanding by the user may be reduced or eliminated, e.g., without the user needing to take initiative, reconfigure the system, etcetera. The system may iteratively adjust the data representation setting and/or data content setting, e.g., as long as the user is confused, angry, irritated, etc. For example, the visualization may be simplified or its type changed until the user understands the way data is visualized, after which details about particular data elements that the user is confused about may be progressively added to provide the user with additional insights. Effectively, the user is guided through the visualization in a way that best matches his cognitive understanding process.

In an embodiment, the user may be a clinician. The data may comprise patient data from multiple modalities and/or comparing the patient to a patient population according to multiple aspects. In such cases, given the time pressure of clinicians and/or the cognitive load they are already placed under due to other tasks, controlling data inspection in a more automatic way according to the needs of the clinician is especially beneficial.

In an embodiment, determining the second data representation setting may comprise determining a globality level of the gaze pattern and a confusion level of the cognitive state and adjusting the first data representation setting if said globality level is high and said confusion level is high. A high globality and a high confusion level may suggest an overall lack of understanding of the way data is represented, such that the user is best helped by an adjustment of the data representation setting, e.g. a simpler or simply another type of visualization.

In an embodiment, determining the second data content setting may comprise determining a globality level of the gaze pattern and a confusion level of the cognitive state and adjusting the first data content setting if said globality level is low and said confusion level is high. If the confusion level is high and the globality level is low, this may suggest that the user is confused about the contents of the visualization, suggesting that the user is best helped by changing the data content setting. For example, a data element of the data may be determined that is relevant to the cognitive state of the user based on the gaze pattern. The first data content setting may then be adjusted based on said data element, e.g., by including additional information about the data element, presenting related data with increased granularity, etc.

In an embodiment, the gaze pattern may comprise multiple points of the visualization on the display and corresponding gaze durations. In such cases, determining the globality level of the gaze pattern may comprise determining an average distance between points of the gaze pattern and/or an average gaze duration. Such values may effectively capture the essential parts of the gaze pattern and may allow for efficient storage and transmission while not losing information necessary to adjust the visualization.

In an embodiment, the gaze pattern sensor comprises a camera for capturing image data of the gaze pattern of the user. Gaze pattern sensing using cameras is known per se and allows to determine gaze patterns relatively cheaply and accurately.

In an embodiment, the cognitive state sensor comprises one or more of a camera for capturing image data of one or more of a facial expression of the user, a gaze pattern of the user, and a pupil dilation of the user; a heart rate sensor; a skin conductance sensor; a mental load sensor, e.g., electroencephalography sensor; and a voice tone sensor. For example, the cognitive state may comprise a confusion level, which can be effectively measured by the above sensors or, even more accurately, by combining their signals.

In an embodiment, adjusting the first data representation setting and/or first data content setting may comprise determining one or more visualization improvement suggestions and determining said setting based on one or more of said visualization improvement suggestions selected by the user. Effectively, a digital system may be obtained that assists the user in finding an optimal representation. In other embodiments, however, no visualization improvements suggestions are selected by the user and instead, the system determines new data representation and/or data content settings directly based on the determined gaze pattern and cognitive state and adjusts the second data representation accordingly without requiring further user interaction.

In an embodiment, the processor may be further configured to determine a cognitive state of the user for the second visualization of the data and to measure whether the choice of the second data content setting and second data representation setting was successful therefrom. This feedback may be used to improve future adjustments to data visualizations, e.g., in the same system or in different systems.

In an embodiment, determining whether said choice was successful may comprise detecting a long gaze away from the visualization and/or long gazes at multiple points of the visualization. Such signals may indicate an understanding of the data by the user and hence a successful adjustment of the data content and/or representation settings.

In an embodiment, the processor may be configured to determine one or more of the first data content setting, the second data content setting, the first data representation setting, and the second data representation setting based on previously measured success of chosen data content settings and data representation settings. This way, the representations are improved in a more effective way since they are based on previous experiences. For example, previous success rates may be used, or a machine learning-trained model suggesting adjustments to the data content and/or representation settings.

An embodiment of the method may be implemented on a computer as a computer implemented method, or in dedicated hardware, or in a combination of both. Executable code for an embodiment of the method may be stored on a computer program product. Examples of computer program products include memory devices, optical storage devices, integrated circuits, servers, online software, etc. Preferably, the computer program product comprises non-transitory program code stored on a computer readable medium for performing an embodiment of the method when said program product is executed on a computer.

In an embodiment, the computer program comprises computer program code adapted to perform all the steps of an embodiment of the method when the computer program is run on a computer. Preferably, the computer program is embodied on a computer readable medium.

Another aspect of the invention provides a method of making the computer program available for downloading. This aspect is used when the computer program is uploaded into, e.g., Apple's App Store, Google's Play Store, or Microsoft's Windows Store, and when the computer program is available for downloading from such a store.

### BRIEF DESCRIPTION OF THE DRAWINGS

Further details, aspects, and embodiments of the invention will be described, by way of example only, with reference to the drawings. Elements in the figures are illustrated for simplicity and clarity and have not necessarily been drawn to scale. In the Figures, elements which correspond to elements already described may have the same reference numerals. In the drawings,
Fig. 1a schematically shows an example of an embodiment of a system for controlling a data inspection by a user,
Fig. 1b schematically shows an example of an embodiment of a system for controlling a data inspection by a user,
Fig. 2 schematically shows an example of an embodiment of a system for controlling a data inspection by a user,
Fig. 3a shows a graph of average distances between points and average gaze durations,
Fig. 3b shows a graph of average distances between points and average gaze durations,
Fig. 4 schematically shows an example of a model of user interaction according to which a data representation setting and/or a data content setting may be adjusted,
Fig. 5a shows an example of a visualization according to a first data representation setting,
Fig. 5b shows an example of a visualization according to second data representation setting,
Fig. 5c shows an example of a visualization according to a second data content setting,
Fig. 5d shows an example of a visualization according to a first data content setting,
Fig. 5e shows an example of a visualization according to second data content setting,
Fig. 6a schematically shows an example of an embodiment of a method of controlling a data inspection by a user,
Fig. 6b schematically shows a computer readable medium having a writable part comprising a computer program according to an embodiment,
Fig. 6c schematically shows a representation of a processor system according to an embodiment.

### List of Reference Numerals:

- 100, 101: a controlling system
- 120: a user
- 110, 111: a data inspection device
- 131: a memory
- 141: a processor
- 151: a communication interface
- 160, 161: a display
- 170, 171: a gaze pattern sensor
- 180, 181: a cognitive state sensor
- 191: a communication network
- 200: a controlling system
- 210: a data inspection device
- 231: data
- 241: a data selection unit
- 242: a data visualization unit
- 243: a visualization selection unit
- 244: a gaze analysis unit
- 245: a cognition analysis unit
- 251: a first data content setting
- 252: a first selection of the data
- 253: a first data representation setting
- 254: a first visualization of the data
- 260: a display
- 261: a second data content setting
- 262: a second selection of the data
- 263: a second data representation setting
- 264: a second visualization of the data
- 265: a gaze pattern
- 266: data indicative of a gaze pattern
- 267: a cognitive state
- 268: data indicative of a cognitive state
- 270: a gaze pattern sensor
- 280: a cognitive state sensor

- 300, 350: average distances between points and average gaze durations

- 410: phase 1: identify high-level structural elements
- 420: phase 2: focus on data elements
- 430, 450: analyzing cognitive state
- 440: phase 3: understanding (gaze away/sequentially)
- 460: adjust data representation setting
- 470: adjust data content setting
- D, Do: average distance, reference distance
- T, To: average time of gaze per point, threshold
- gz. awy./seq.: gaze away/sequential reading

- 530: additional information about a data element
- 531: a data element

- 900: a controlling method
- 910: storing data
- 920: showing a first visualization
- 930: sensing data indicative of a gaze pattern
- 935: determining the gaze pattern
- 940: sensing data indicative of a cognitive state
- 945: determining the cognitive state
- 950: determining second data content and representation settings
- 960: showing a second visualization of the data

- 1000: a computer readable medium
- 1010: a writable part
- 1020: a computer program

- 1110: integrated circuit(s)
- 1120: a processing unit
- 1122: a memory
- 1124: a dedicated integrated circuit
- 1126: a communication element
- 1130: an interconnect
- 1140: a processor system

### DETAILED DESCRIPTION OF THE EMBODIMENTS

While this invention is susceptible of embodiment in many different forms, there are shown in the drawings and will herein be described in detail one or more specific embodiments, with the understanding that the present disclosure is to be considered as exemplary of the principles of the invention and not intended to limit the invention to the specific embodiments shown and described.

In the following, for the sake of understanding, elements of embodiments are described in operation. However, it will be apparent that the respective elements are arranged to perform the functions being described as performed by them.

Further, the invention is not limited to the embodiments, and the invention lies in each and every novel feature or combination of features described herein or recited in mutually different dependent claims.

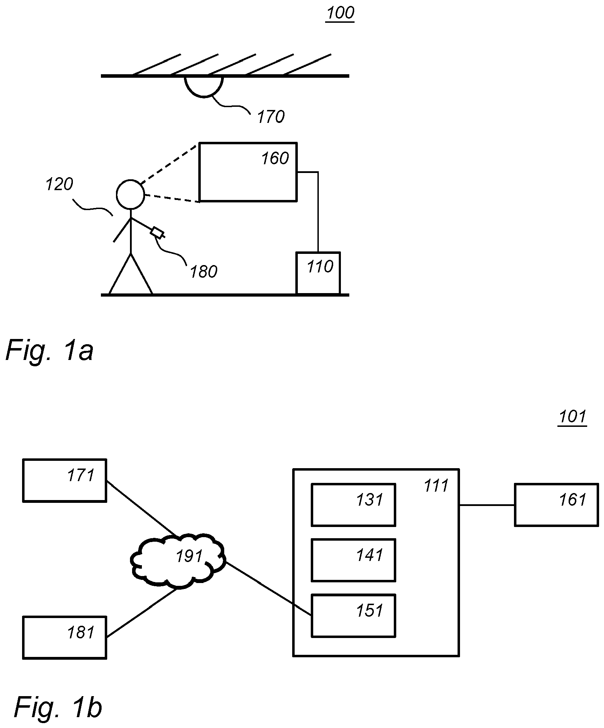

**Fig. 1a** schematically shows an example of an embodiment of a system 100 for controlling a data inspection by a user 120. As an illustrative example, system 100 is here installed in a room, e.g., system 100 may be installed in a meeting room, for example, a tumor board meeting room arranged for a radiologist to perform a patient assessment.

System 100 may comprise a display 160 for showing a visualization of the data to be inspected. Display 160 may be any kind of device for presentation of information in visual form, e.g., a television screen, a computer monitor, a projection system, etc. For example, display 160 may be a projection system comprising a projector, e.g., a beamer, and a surface, e.g., a screen, on which images projected by the projector are displayed. Display 160 can be integrated with other parts of system 100, e.g., display 160 can be a screen of a device, e.g., tablet or smartphone, that may also implement various other parts of the system, e.g., display 160 may be integrated in data inspection device 110 discussed below. Displays are also known as display devices, electronic displays, electronic visual displays, etc. In the example shown here, display 160 is mounted on a wall of the room in which system 100 is installed.

The visualization on display 160 may be shown to a user 120. In various embodiments, user 120 is a clinician, e.g., a radiologist. The data to be visualized may comprise patient data. The system may for instance be arranged for clinician 120 to perform a patient assessment, e.g., to decide on a further course of treatment or to perform a diagnosis of the patient. Such decisions may need to be taken in limited time. Moreover, patient data may be relatively complex data, e.g., the data may comprise patient data from multiple modalities and/or data comparing the patient to a patient population according to multiple aspects. However, the invention is by no means limited to patient data and various other examples of data inspections are provided throughout.

System 100 may also comprise a memory configured to store the data to be inspected, and a processor. In this example, the memory and processor are integrated into a data inspection device 110. Various other arrangements of processor and memory are possible, e.g., at least some of the operations performed by the processor may be performed in remotely or using cloud computing. The processor of inspection device 110 may be configured to show, e.g., on display 160, a first visualization of the data. The visualization may comprise a visualization according to a first data representation setting of a selection of the data according to a first data content setting. For example, the first data content setting may be indicative of what data to show to user 120. According to this first data content setting, a selection of the data may be made. The first data representation setting may be indicative of how to show this selected data. Various examples are provided herein.

Interestingly, system 100 may track in various ways how user 120 perceives the visualization shown on display 160. In particular, system 100 may comprise a gaze pattern sensor 170. Gaze pattern sensor 170 may be for sensing data indicative of a gaze pattern of the user 120 who is looking at the visualization shown on display 160, for example, the first visualization as described above. Based on this sensed data, the processor of system 100 may determine the gaze pattern of user 120 for the first visualization of the data. Shown in the figure is a camera 170 for capturing image data of the gaze pattern of the user. As shown in the figure, camera 170 may be mounted in a room in which system 100 is installed. Camera 170 can also be integrated with other parts of the system, e.g., display 160 and camera 170 can both be part of the same device, e.g., a smartphone or tablet. Instead of or in addition to cameras, various other kinds of known gaze pattern sensors may be used.

System 100 may also comprise a cognitive state sensor 180. Cognitive state sensor 180 may be for sensing data indicative of a cognitive state of user 120 looking at the visualization shown on display 160, for example, the first visualization. Based on this sensed data, the processor of system 100 may determine the cognitive state of user 120 for the first visualization of the data. The cognitive state may be indicative of whether a clarification of the shown visualization is needed. In an embodiment, the cognitive state comprises a confusion level of user 120, e.g., data indicating whether user 120 is confused and/or an extent of confusion of the user 120. A confusion level may also indicate, instead or in addition, an amount of understanding of the user 120. The cognitive state sensor 180 may in various embodiments coincide partially or completely with the gaze pattern sensor 170, e.g., both may be or comprise the same camera.

In the figure, cognitive state sensor 180 comprises a heart rate sensor worn by the user 120, for example as an armband or as a component of a smart watch. However, various other known types of cognitive state sensors may be used with corresponding techniques to determine the cognitive state of the user from their sensor data. For example, in various embodiments, the cognitive state sensor 180 may comprise a skin conductance sensor. For example, increased conductance may be indicative of arousal and/or of higher confusion level of user 120. In various embodiments, the cognitive state sensor 180 may also comprise a mental load sensor, for example, a sensor for performing electroencephalography (EEG) measurements of the user. For example, increased mental load may be indicative of confusion. Cognitive state sensor 180 may also comprise a voice tone sensor, e.g., a microphone, e.g., for performing voice tone analysis in order to determine the cognitive state of the user. Data from multiple sensors, e.g., a camera and a heart rate sensor, may be combined to obtain a more reliable measurement of the cognitive state, e.g., a confusion level may be determined as a maximum, average, minimum, etc. of multiple sensor measurements.

In various embodiments, cognitive state sensor 180 may comprise a camera, e.g., the same camera as gaze pattern sensor 170. The camera may capture image data of one or more of a facial expression of user 120, a gaze pattern of user 120, a pupil dilation of user 120, and a head movement of user 120. For example, detecting confusion levels from facial expression images and/or pupil dilation may be performed using techniques known in the art per se. Detecting a confusion level based on a gaze pattern of the user may comprise determining whether the user is looking at the visualization on display 160. For example, if user 120 is looking away from the visualization for a relatively long time, this may indicate an understanding of the visualization. If the user is looking at a part of the visualization, this may indicate that the user is confused by a particular part of that visualization. In particular, it was a realization of the inventors that a gazing back and forth with a decreasing number of items per amount of time is indicative of a confusion of the user with the global contents of a shown visualization. As another example, head movements or positions of user 120 may be used as an indication of confusion, e.g., detection of a tilting movement or position could be used as an indication that the user has a problem to understand parts of the visualization, e.g., labels were hard to read. Similarly, an uncontrolled head movement may indicate problems understanding the visualization, e.g., confusion, as well.

The processor of system 100 may be configured to determine, based on the determined gaze pattern and cognitive state, a second data content setting, e.g., indicative of what data to show, and a second data representation setting, e.g., indicative of how to show this data. Interestingly, system 100 is not configured to always only adjust the data content setting or to always only adjust the data representation setting. Instead, for at least a first set of determined gaze patterns and cognitive states, the system adjusts the data content setting and for at least a second set of determined gaze patterns and cognitive states, the system adjusts the data representation setting. The first and second sets may be disjoint, e.g., in some embodiments, the system adjusts either the data content setting or the data representation setting, but this is not necessary. The system could also, sometimes or always, adjust both the data content setting and the data representation setting at the same time.

In any case, the processor of system 100 may show a second visualization of the data, comprising a visualization according to the second data representation setting of a selection of the data according to the second data content setting, e.g., on display 160. This process may be repeated, e.g., also a gaze pattern and cognitive state may be determined for the second visualization, based on which a data content setting and a data representation setting are determined for a third visualization that is then shown, etcetera. For example, the process of determining and showing visualizations may be repeated until the data sensed by the cognitive state sensor indicates that user 120 is no longer confused.

Because system 100 does not always only adjust the data content setting or always only adjust the data representation setting, better second or subsequent visualizations are shown to user 120, allowing more efficient controlling of the data inspection being performed. For example, system 100 is able to more quickly adapt the visualization according to the user's needs. If the data content needs to be changed, the second data visualization may be based on an updated data content setting. Instead or in addition, if the data representation needs to be changed, the second data visualization may be based on an updated data representation setting. No user input interface, e.g., keyboard, mouse or touch screen, may be required to control the data inspection, e.g., the user may not need to use his hands. Thereby, it becomes easier to perform the data inspection when the user needs his hands for other tasks, e.g., to perform a surgery or to control another data visualization.

For example, in various embodiments, user 120 is a clinician who, alongside the data inspection by system 100, performs other tasks such as performing an operation on the patient or inspecting a 3D visualization of the patient, e.g., using a joystick or keyboard. For example, the visualizations of system 100 may visualize patient data related to the other task that user 120 is performing, e.g., the visualization may show vital signs of the patient, measurements concerning the part of the body where the surgery is taking place or which is visualized in the 3D visualization, etcetera. In such embodiments, the user is able to control data inspection while at the same time performing the other tasks, e.g., a task for which user 120 needs to use hands.

In the particular example shown in Fig. 1a, a single user 120 is shown. This is not a limitation in that system 100 can also support multiple people. For example, system 100 can support multiple people looking at respective visualizations for which respective data content and data representation settings are determined. Interestingly, for at least some of the people, the same gaze pattern sensor and/or the same cognitive state sensor may be used, increasing efficiency. The system can also work for multiple people looking at the same data visualization. In this case, for example, the data content setting and/or data representation setting can be adjusted based on gaze patterns and cognitive states of the multiple users, e.g., based on deciding adjustments that are best for a majority of users, etc. The system may also recognize one main user and determine the adjustments based on the gaze pattern and cognitive state of just that user.

In this particular example, one display 160 is shown for showing a single visualization of the data. The system can also be arranged to show multiple visualizations to user 120, e.g., on the same display 160 or on multiple displays. In this case, the system can for instance be arranged to update the data content setting and the data representation setting for a particular visualization if the gaze pattern of the user indicates that the user is looking at that particular visualization.

**Fig. 1b** schematically shows an example of an embodiment of a system 101 for controlling a data inspection by a user. For example, system 101 may be based on system 100. System 101 may comprise a display 161 for showing a visualization of the data, e.g., based on display 160. System 101 may comprise a gaze pattern sensor 171 for sensing data indicative of a gaze pattern of the user looking at the visualization shown on display 161, e.g., based on gaze pattern sensor 170. System 101 may also comprise a cognitive state sensor 181 for sensing data indicative of a cognitive state of the user looking at the visualization shown on display 161, e.g., based on cognitive state sensor 180.

System 101 may also comprise a data inspection device 111. Data inspection device 111 may comprise a memory 131 and a processor 141. Memory 131 may be used for data and/or instruction storage. For example, memory 131 may comprise software and/or data on which processor 141 is configured to act. Memory 131 may also store the data to be visualized. Processor 141 may be implemented as one or more processor circuits, e.g. microprocessors, ASICs, FPGA and the like. Memory 131 may comprise computer program instructions which are executable by processor 141. Processor 141, possibly together with memory 131, may be configured according to an embodiment of a data inspection device. Interestingly, data inspection device 111 in some embodiments does not comprise a user input interface for obtaining user input apart from the data sensed by the gaze pattern sensor and cognitive state sensor, or at least, no such input interface may be used by data inspection device 111 to determine settings for the second visualization.

Data inspection device 111 may also comprise a communication interface 151 configured for digital communication with at least gaze pattern sensor 171 and cognitive state sensor 181. The gaze pattern sensor and/or cognitive state sensor 181 may comprise corresponding communication interfaces arranged for digital communication with data inspection device 111 (not shown). Communication interface 151 may be arranged for direct communication with the respective devices, e.g., using USB, HDMI, or similar interfaces. Communication interface 151 may also communicate over a computer network 191. Computer network 191 may be a wireless personal area network, e.g., using ZigBee, IrDA, wireless USB, Bluetooth, or similar. However, computer network 191 may also be another type of network, e.g., an internet, an intranet, a LAN, a WLAN, etc. For instance, communication interface 151 may comprise a connector, e.g., a wireless connector, an Ethernet connector, a Wi-Fi, 4G or 4G antenna, a ZigBee chip, etc., as appropriate for computer network 191. Computer network 191 may comprise additional elements, e.g., a router, a hub, etc. Data inspection device 111 may comprise a display interface for outputting data visualizations to display 161, e.g., data visualizations may be output directly to display 161 or indirectly, e.g., data inspection device 111 may determine an image or other descriptor of a data visualization for visualization by on display 161.

Processor 141 may be configured to show a first visualization of the data, e.g., on display 161, comprising a visualization according to a first data representation setting of a selection of the data according to a first data content setting.

System 101 may be configured to determine the gaze pattern of the user for the first visualization of the data based on the data sensed by gaze pattern sensor 171. For example, gaze pattern sensor 171 may itself be configured to determine the gaze pattern and provide it to data inspection device 111. Alternatively, data inspection device 111 may be configured to receive the sensed data from gaze pattern sensor 171 and, using processor 141, to determine the gaze pattern therefrom.

System 101 may also be configured to determine the cognitive state of the user for the first visualization of the data based on the data sensed by cognitive state sensor 181. For example, cognitive state sensor 181 may be configured to determine the cognitive state and provide it to data inspection device 111. Alternatively, data inspection device 111 may be configured to receive the sensed data from cognitive state sensor 181 and, using processor 141, to determine the cognitive state therefrom.

Processor 141 may be configured to determine, based on the gaze pattern and the cognitive state, a second data content setting and a second data representation setting. Processor 141 may be further configured to show a second visualization of the data, e.g., on display 161, comprising a visualization according to the second data representation setting of a selection of the data according to the second data content setting.

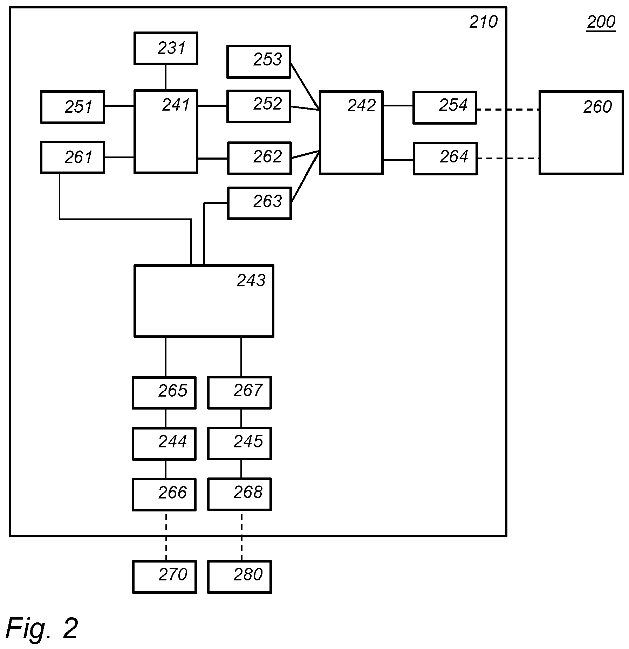

**Fig. 2** schematically shows an example of an embodiment of a system 200 for controlling a data inspection by a user. System 200 may comprise data inspection device 210, display 260, gaze pattern sensor 270, and cognitive state sensor 280. For example, the system and its components may be based on system 100 or 101 and components 110 or 111, 260 or 261, 270 or 271, and 280 or 281, respectively. Fig. 2 schematically shows functional units that may be functional units of a processor of data inspection device 210, e.g., processor 141 of data inspection device 111. For example, Fig. 2 may be used as a blueprint of a possible functional organization of the processor. For example, the functional units shown in Fig. 2, e.g., the data selection unit 241, may be wholly or partially be implemented in computer instructions stored at device 210, e.g., in an electronic memory of device 210, and are executable by a microprocessor of device 210. In hybrid embodiments, functional units are implemented partially in hardware, e.g., as coprocessors, and partially in software stored and executed on the device.

Data inspection device 210 may comprise various units arranged to produce visualizations of data 231 stored in its memory. Data 231 may comprise one or more of tabular data, image data, video data, numeric data, textual data, etc. Data inspection device 210 may produce visualizations of the data according to respective data content settings, e.g., indicating what data to select for visualization, and data representation settings, e.g., indicating how to visualize the selected data. For example, shown in the figure are a first data content setting 251 and a first data representation setting 253. First data content setting 251 and/or first data representation setting 253 may be default settings, e.g., they may be predefined or user-selected.

In order to produce visualizations of the data, data inspection device may comprise a data selection unit 241. Data selection unit 241 may be configured to make selections of data 231 according to respective data content settings, e.g., first data content setting 251 or second data content setting 261 discussed later. This leads to respective selections of the data, e.g., first selection 252 of the data and second selection 262 of the data, respectively. For example, the selection of data may comprise a filtering, a sampling at a given granularity, an image filter such as a cropping or a scaling, etcetera.

In various embodiments, data 231 comprises a first data portion to be visualized and a second data portion comprising additional information about one or more data elements of the first data portion. For instance, the first data portion may comprise one or more portions of text or other data to be shown, and the second data portion may comprise additional information about one or more parts, e.g., words or phrases, of the first portion, e.g., definitions, pronunciations, synonyms or data that is otherwise correlated to the first portion. The first data portion may also comprise one or more data points, e.g., geographically or temporally distributed data points, whereas the second data portion contains additional information, e.g., a data series per data point along another distribution. For example, if the first data points are geographically distributed, e.g., occupation levels of hospital rooms, then the data series about the first data point may be temporally distributed, e.g., development of the occupation level of a given room over time, or the other way around.

In such cases, data selection unit 241 may obtain a selection of zero or more data points, and perform its data selection by adding, to the first data portion of data 231, additional information from the second portion about the selected data points of the first data portion. For example, in first data content selection 251, no such data points may be selected but in second data content selection 252, one or more data points may be selected based on user feedback, as discussed below.

Various other types of data selection to be performed by data selection unit 241 will be apparent. Such data selections need not be based on providing additional information on particular data elements from the visualization that are singled out. Generally, changing a data content selection typically implies showing different data to the user, e.g., although its representation may be the same or similar. This way, if the user understands the representation but is interested in seeing different data, this can be facilitated by adjusting the data content selection. For instance, a data content selection may comprise a selection of a particular subset of data 241, e.g., a particular region of a graph that the user is particularly interested in.

In various embodiments, data content selections 251, 261 may comprise an amount of data to be displayed and/or a granularity at which to display data 231. In such cases, data selection unit 241 may filter data 231 accordingly.

It is not necessary that data content selections 251, 261 lead to overlapping sets 252, 262 of data, e.g., a user with an interest in a particular item selected by data content selection 251 may want to see a different kind of information about that particular item that was not previously selected in selection 252. Various alternatives will be apparent to the skilled person and illustrative examples are provided below.

Data inspection device 210 may further comprise a data visualization unit 242. Data visualization unit 242 may be configured to determine a visualization according to respective data representation settings of selections of data 231. For example, in various embodiments, data visualization unit determines a first visualization 254 of first selection 252 based on first data representation setting 253 and a second visualization 264 of second selection 262 based on second data representation setting 263 as also discussed below. It is known that for given data 252, 262 to be visualized, various types of visualization are possible, e.g., a bar chart or a line chart, a textual or a visual representation, etc. Moreover, multiple data series may be visualized in a combined representation, e.g., a graph showing the multiple data series, or in separate representations, e.g., multiple graphs showing the respective data series separately, depending on the data representation setting. Accordingly, each of visualizations 262, 254 may in various embodiments comprise one or more graphs, infographics, maps, tables, dashboards, animations and/or interactive multimedia visualizations, text visualizations, etcetera. Various examples are provided throughout. Moreover, a visualization of a given type may be customizable by one or more settings, e.g., a font size setting or an item ordering setting. Data visualization unit 242 may obtain a type of visualization and/or one or more settings for the visualization type and determine a visualization 254, 264 of respective data 252, 262 accordingly. Respective visualizations 254, 264 may be shown to the user, e.g., on display 260.

For example, data representation settings 253, 263 may comprise a type of visualization used to visualize the respective selections 252, 262, of the data. For instance, the type of visualization can indicate whether to visualize the data by a table or by a particular type of graph, e.g., a line chart, a bar chart, a map showing spatially or geographically distributed data, etc., and/or how to visualize data in a particular type of visualization, e.g., data representation settings 253, 263 can comprise one or more of a color setting, a font size setting, a text spacing setting, a geometry setting, and an item order setting. For example, in a 2D graph, data representation setting 253, 263 can indicate which quantity to visualize along a first axis and which quantity to visualize along a second axis, and similarly for 3D graphs. As is known from the literature, for instance in the context of A-B testing of visualizations, the same data 252, 262 can be visualized in various ways and by using a data representation that best fits the user looking at the visualization, better understanding is obtained.

Data inspection device 210 may further comprise a gaze analysis unit 244. As discussed with respect to Fig. 1b before, data inspection device 210 may be configured to obtain data 266 indicative of a gaze pattern from gaze pattern sensor 270. Gaze analysis unit 244 may be configured to determine a gaze pattern 265 of the user for first visualization 254 of data 231 based on this data 266. Alternatively, data inspection device 210 may directly obtain gaze pattern 265 from the gaze pattern sensor 270, in which case a gaze analysis unit may be unnecessary. Typically, gaze pattern 265 comprises multiple points of the visualization on the display and corresponding gaze durations. Various techniques to determine such gaze patterns, e.g., using a camera, are known in the art and may be readily applied.

Data inspection device 210 may also comprise a cognition analysis unit 245. As discussed with respect to Fig. 1b, data inspection device 210 may obtain data 268 indicative of a cognitive state of the user from cognitive state sensor 280, based on which the cognition analysis unit may determine cognitive state 267 of the user for first visualization 254 of data 231. Data inspection device 210 can also obtain cognitive state 267 directly. Various possible cognitive state sensors 280 have been discussed above. In particularly advantageous embodiments, cognitive state 267 comprises a confusion level of the user.

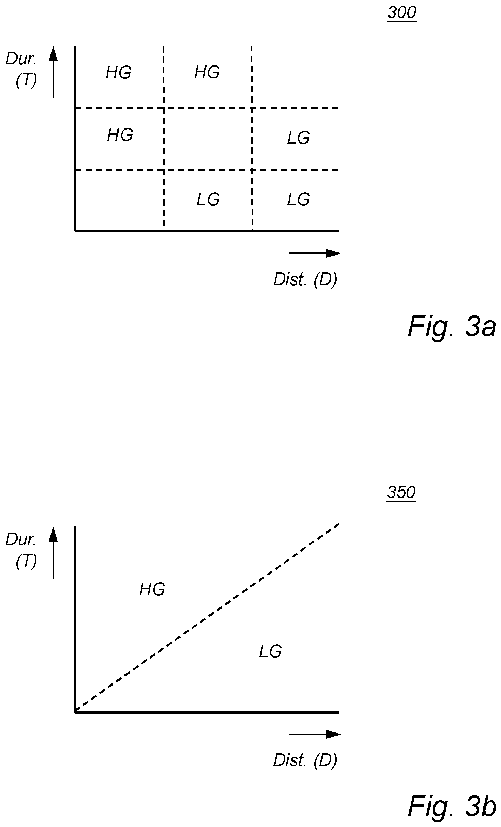

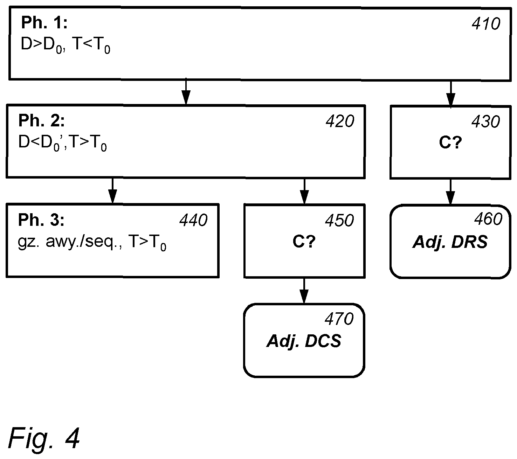

Data inspection device 210 may also comprise a visualization selection unit 243. Visualization selection unit 243 may be configured to determine second data content setting 261 and second data representation setting 263 based on gaze pattern 265 and cognitive state 267. In various embodiments, visualization selection unit 243 may determine a globality level of gaze pattern 265. The globality level may be indicative of whether the user is looking globally or locally at the visualization. The globality level can be continuous, e.g., a value between 0 and 1, or discrete, e.g., global/intermediate/local, high/low, etc. Determining the globality level of gaze patterns can be done, e.g., as discussed with respect to Figs. 3a, 3b and 4. Visualization selection unit 243 may also determine a confusion level of cognitive state 267. For example, the confusion level may be continuous, e.g., a value between 0 and 1, or discrete, e.g., confused/not confused.

Generally, visualization selection unit 243 may adjust the data content setting and/or data visualization setting if the user is confused, e.g., if the cognitive state 267 indicates confusion e.g., the determined confusion level exceeds a predefined threshold. On the other hand, if the user is not confused, no adaptation of the visualization may be needed. Interestingly, whether or not to adjust data content setting 251 and whether or not to adjust data representation setting 253 may both be decided based on the determined globality level. For example, for at least some values of the determined globality level, the data content setting may be adjusted and for at least some values of the determined globality level, the data visualization may be adjusted. This may enable improving the visualization in a more flexible way that is better adjusted to the user's cognitive needs and gives faster understanding of the data. In some embodiments, visualization selection unit 243 may adjust the data content setting or the data representation setting, but not both. This may make the cognitive task for the user to interpret the second visualization 264 easier since the user still sees either the same data or the same type of visualization.

In some embodiments, visualization selection unit 243 is configured to adjust first data representation setting 253 if the globality level is high and the confusion level is high. In other words, second data representation setting 263 may in such cases be modified from first data representation setting 253. Otherwise, visualization selection unit 243 may for example set second data representation setting 263 to be equal to first data representation setting 253. Visualization selection unit 243 may determine that the globality level is high and the confusion level is high in various ways. For example, visualization selection unit 243 may compare both levels to respective predefined thresholds, or visualization selection unit 243 may evaluate a function that is increasing in both the globality level and the confusion level and compare, for example, the result of that function evaluation to a predefined threshold.

Adjusting the first data representation setting 253 may comprise simplifying the visualization of the data, or at least choosing a different visualization in such a way that the user may find it easier to understand how the data is represented. For example, a different data representation setting may be chosen from a predefined list, randomly, etc. In other embodiments, adjusting the first data representation setting 253 comprises determining second data representation 263 to or based on an output of a rule-based engine, e.g., based on cognitive state measurements for the first data representation setting and/or other previous data representation settings. Various examples are provided below.

By adjusting the first data representation setting 253 if the globality level is high and the confusion level is high, controlling system 200 may be able in various embodiments to present a second visualization 264 that is better adjusted to the cognitive needs of the user in that situation, e.g., by presenting a simpler or at least different visualization if the user is found not to sufficiently understand the way data is visualized in visualization 254.

In some embodiments, visualization selection unit 243 is configured to adjust first data content setting 251 if the globality level is low and the confusion level is high. In other words, second data content setting 261 may be modified in this case from first data content setting 251; otherwise, visualization selection unit 243 may for example set second data content setting 261 to be equal to first data content setting 251 or not touch the data content setting at all. Analogously to the data representation setting, visualization selection unit 243 may for example compare the globality level and the confusion level to predefined thresholds, compute a function that is increasing in the confusion level and decreasing in the globality level, etc.

In various embodiments, if the confusion level is high, then visualization selection unit 243 adjusts the first data representation selection if the globality level exceeds a threshold and otherwise adjusts the first data content selection. However, this is not necessary, for example, visualization selection unit 243 may also determine that the globality level is neither low nor high, in which case neither the data content setting nor the data representation setting may be modified, the user may be asked for feedback, etc.

Adjusting the first data content setting 251 may comprise including information that enables the user to better understand what data is being visualized. In various embodiments, visualization selection unit 243 adjusts first data content setting 251 based on one or more particular data elements of visualization 254 relevant to the cognitive state of the user. The data element may for example be a data point or a data series in a graph, an axis, a particular portion of a text such as a word or a phrase, etcetera. For example, the user may have been looking at the particular data element at the same time or just before the cognitive state 267 of the user was associated with a particular cognitive state such as confusion or frustration. Visualization selection unit 243 may determine the particular data element from gaze pattern 265. Adjusting first data content setting 251 in such cases may comprise including additional information about this data element, for example, in the form of text, in the form of more fine-grained data concerning the data element, e.g., geographically partitioned data using a finer partitioning, etcetera. In various embodiments, the additional information is presented in a pop-up or next to the visualization.

However, the adjustment to first data content setting 251 need not be specific to any particular data elements. For example, the user may understand the way data is visualized but may not realize a pattern exhibited by the data, e.g., a correlation or temporal relation of data shown in the visualization. In such cases, adjusting first data content setting 251 can, for example, comprise including additional information about such a pattern exhibited by the data, e.g., in the form of text, in the form of an additional graph, etcetera. Various examples of adjusting the data content setting are provided below.

By adjusting the first data content setting 251 if the globality level is low and the confusion level is high, controlling system 200 may be able in various embodiments to present a second visualization 264 that is better adjusted to the cognitive needs of the user in that situation, e.g., by presenting information that allows the user to better understand the information in visualization 254 that the user was confused about.

In either case, as discussed above, data selection unit 241 may be configured to make a selection 262 of data 231 according to second data content setting 261 and data visualization unit 242 may be configured to determine second visualization 264 of selected data 262, analogously to first visualization 234. Visualization 264 may be shown, e.g., on display 260.

Various aspects of controlling system 200 are now further elaborated upon below with reference to Fig. 3a-5e.

In various embodiments, visualization selection unit 243 determines the globality level of gaze pattern 265 based on an average distance between points of the gaze pattern and/or an average gaze duration. The average distance between points may be expressed, for example, in terms of an absolute length, e.g., centimeters; or as a relative length, e.g., a percentage of a total data visualization dimension or a number of times the distance between closest data elements of the visualization. The average duration may similarly be measured absolutely, e.g., in seconds; or relatively, e.g., as percentage of the total time spent looking at the visualization.

Various ways in which the globality level may be determined are now explained with reference to the graphs shown in **Fig. 3a** and **Fig. 3b****.** In various embodiments such as those exemplified by these figures, visualization unit 243 may determine that the globality level is a high globality level or a low globality level. Visualization unit 243 may also, for example, determine that the globality level is intermediate. Visualization unit 243 may also determine the globality level as a continuous variable, e.g., by dividing the gaze duration by the average distance, etc. Generally, higher globality levels are associated with shorter average gaze durations and/or higher average distance between points.

Fig. 3a shows a plot of possible values of the average distance between points of the gaze pattern, shown here along the x-axis labelled "Dist. (D)" against possible values of the average gaze duration, shown here along the y-axis labelled "Dur. (T)". As shown here, visualization selection unit 243 may classify the average distance to be low, intermediate, or high, for example, based on predefined thresholds. For example, the average distance may be low if it is at most 20% of the total data visualization dimension, e.g., the diagonal dimension of the visualization. The average distance may be high, for example, if it is at least 30% of the total data visualization dimension. Visualization selection unit 243 may also classify the average gaze duration to be low, intermediate, or high, for example, based on predefined thresholds, e.g., at most 0.5 seconds for low duration and at least 1 second for high duration.

As denoted in the figure as "HG", unit 243 may for instance determine a high globality at least for high duration with low distance, and optionally also for high duration with intermediate distance and/or intermediate duration with high distance. As denoted in the figure as "LG", unit 243 may for instance determine a low globality at least for low duration and high distance, and optionally also for low duration and intermediate distance and/or intermediate duration and high distance. In some embodiments, high duration with high distance and low duration with low distance may not lead to either classification, although in other embodiments, such a case could be classified in either way. It is not necessary for the intermediate categories to exist, e.g., distance may be classified as high or low, e.g., higher or lower than 30% of the total visualization dimension, and/or duration may be classified as high or low, e.g., higher or lower than one second.

Fig. 3b shows another plot of possible values of average distance, "Dist. (D)" versus average gaze duration, "Dur. (T)". As shown here, visualization selection unit 243 may determine a low globality, shown as "LG", and/or a high globality, shown as "HG", based on one or more decision boundaries. Generally, the higher the average distance and the lower the average duration, the sooner the globality is high. In other words, the higher the duration, the higher average distance may be for globality to high, and vice versa. For instance, the decision boundary may be given by average distance minus average duration, for appropriately scaled average distance and duration, as shown by this figure; by average distance divided by average duration; etcetera. Although not shown in the figure, globality may also be classified as neither low nor high, e.g., as an intermediate globality.

**Fig. 4** schematically shows an example of a model of user interaction according to which a data representation setting and/or a data content setting may be adjusted, e.g., by visualization selection unit 243 of Fig. 2. Based on an average distance between points of gaze pattern 265 and an average gaze duration, visualization selection unit 243 may identify whether the user analyses the structure or the content of visualization 254, and based on this, adjust data representation setting 253 and/or data content setting 251 accordingly. In other words, in case the user is confused, visualization selection unit may determine whether the user is confused by the graphical representation of the data or by the content of the data. This determination may trigger actions of the dynamic visualization to optimize the graphical representation, by adjusting data representation setting 253, or the data content, by adjusting data content setting 251.

Based on analysis of gaze patterns for a variety of graphs, infographics, etcetera, the inventors have identified a common pattern for gaze patterns of users. Specifically, the inventors recognized that users may pass through one or more of the following phases shown in the figure:
In **Phase 1,** 410, the user may identify high-level structural elements of the visualization, e.g. title, geometry, axis position and axis unit of a graph. This phase may be characterized by a relatively high average distance *D* between points gazed at, e.g., at least a reference distance, e.g., a threshold such as *D₀*=30% of the total data visualization dimension, and/or by a relatively low average time T of gaze per point, e.g., at most a reference time, e.g., a threshold such as *T₀*=1 second.
   In Phase 1, based on analyzing, 430, the cognitive state 267 of the user, visualization selection unit 243 may adjust, 460, data representation setting 253. For example, if the cognitive state indicates confusion and/or frustration, data representation setting 253 may be adjusted, e.g., to simplify the visualization. The inventors have found that, in addition to other discussed techniques such as facial expression analysis, detecting a gazing back and forth with a decreasing number of items per amount of time can also be a good indication for confusion in this case.
In **Phase 2,** 420, the user may look with longer fixation at various data elements of the visualization, e.g. axis coordinates, graph content like lines/bars/dots, legend text, and relations between such data elements. This phase may be characterized by a relatively low average distance *D* between points gazed at, e.g., at most once or twice reference distance *D₀*' between the closest similar item, and/or a relatively long average time *T* per point, e.g., at least *T₀*=one second.
   In Phase 2, based on analyzing, 450, the cognitive state 267 of the user, visualization selection unit 243 may adjust 470, data content setting 251. For example, cognitive state 267 may indicate confusion or frustration. For example, additional information can be included about a particular element of the visualization that the user was looking at.
In **Phase 3,** 440, when understanding the data, the user may read through the data items one by one and in order, e.g. down a list. In some cases, after having read data elements one by one, a user may go back to read a few items read previously, e.g., the most interesting ones, to process the content and draw conclusions. This phase may be characterized, for instance, by a long gaze away from the visualization, e.g., time *T* at least *T₀*=1 second, and/or sequential reading of a list of data elements and going back to read a number of particular items, e.g., one or two items. Pupil dilation may also indicate the user entering this phase.

Visualization selection unit 243 may use detection of phase 3, for example, as feedback that the current data representation setting 253, 263 and/or data content setting 251, 253 used for visualizations 254, 264 are successful, e.g., such feedback may be stored for improving future data representation/content settings, as also discussed below.

It is noted that this viewing pattern applies to a broad range of viewers, e.g., the phases described herein may relate to general viewing patterns beyond individual variability.

It is not necessary for a user to go through each of the phases, e.g., a user may skip Phase 1, 410, and directly enter Phase 2, 420 or Phase 3, 440. For example, a user who has previously seen the same type of visualization of different data may need less or no time to understand the global structure.
A number of representative examples of observed viewing patterns are the following:

| **Example 1 (no confusion).** |
|---|
| - Phase 1, understand global structure: large gaze movements, look at general shape and various types of labels |
| - Phase 2, data analysis: read a few specific labels one by one, look at data subdivisions |
| - Phase 3, deeper dive in data: read labels one by one, slower. |

| **Example 2 (no confusion).** |
|---|
| - Phase 1, understand global structure: look quickly at data series, at axis high-level content |
| - Phase 2, data analysis: data labels, check correspondence with axis coordinates |
| - Phase 3, understand data: read text next to data series in order, possibly go back to most interesting data series |

In the following examples, confusion arose, e.g., the viewer never reaches phase 2 and/or the viewer during phase 1 does a lot of quick back and forth between items.

| **Example 3 (confusion).** |
|---|
| - Phase 1: global eye movements: look at graph components and labels, followed by frowning |
| and quick back-and-forth between parts of graph, uncontrolled head movements, etc. |
| - Phase 2 is not reached, suggesting bad graphical representation |

| **Example 4 (confusion).** |
|---|
| - Phase 1: random gaze at data elements, gaze faster and faster |
| - Phase 2 is not reached, suggesting bad graphical representation |

| **Example 5 (confusion).** |
|---|
| - Phase 1, understand global structure: global gaze movements on data elements and corresponding colors in legend, |
| - Phase 2: read legend text, look at corresponding colors on card, frowning, content not understood. |

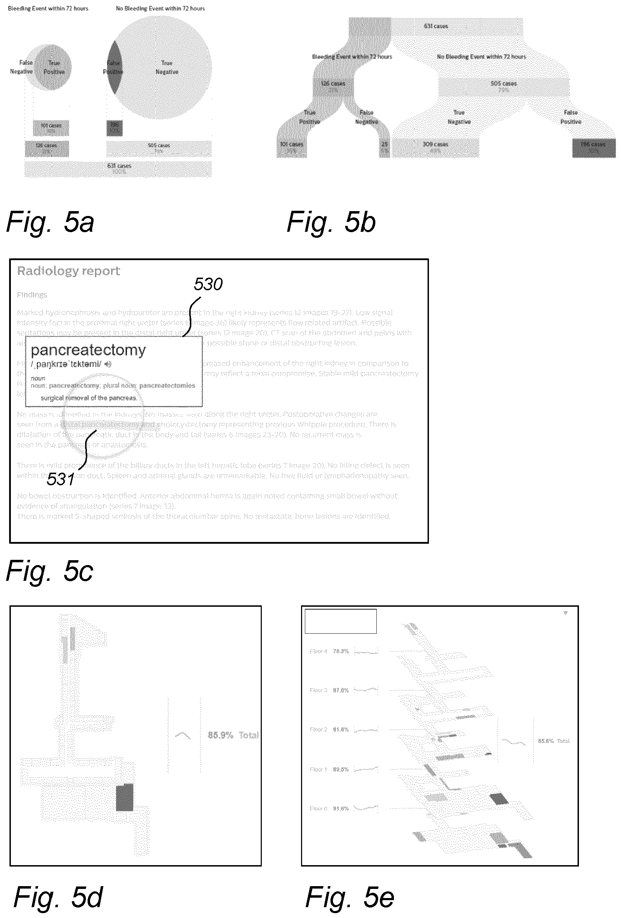

Various ways in which visualization selection unit 243 may adjust the data content setting 251 and/or data representation setting 253 are now discussed with respect to the example visualizations of **Fig. 5a-5e****.**

Adjusting data representation setting 253, e.g., in response to a detected confusion or frustration of the user, may comprise simplifying data representation setting 253 or adjusting the data representation setting in the expectation that the user may find it easier to understand. For example, a visual format and/or parameter of a type of visualization may be varied. As an example, confusion may be detected for the relatively complex visualization shown in **Fig 5a****.**

As a response, visualization selection unit 243 may choose the different representation of **Fig. 5b** that may be considered easier to understand. Various simplifying measures include decreasing a granularity of the visualization, e.g. moving from a daily view to a weekly view, increasing a font size, etcetera. Visualization selection unit 243 may try out different representations of the data until it is detected that the user understands the visualization, e.g. enters phase 2 or 3 and/or the confusion level of the user drops below a threshold. In case of a simplification, visualization selection unit 243 may be configured to, upon detecting that the simplified visualization is understood by the user, at least partly cancel the simplification, e.g., by reverting to the original visualization or gradually adding back details as long as it is indicated that they are understood by the user. The additional information may also be made available to the user, e.g., in the form of a popup.

As also highlighted in Fig. 5a and Fig. 5b, the second data representation setting 263 does not need to represent a simplification in the sense that less data is shown, e.g., the same data can also be represented in a different way, e.g., by splitting a combined graph showing multiple data series at the same time into multiple graphs.

As discussed above, visualization selection unit 243 may instead or in addition adjust data content selection 251, e.g., in case of a low globality of the gaze pattern 265. For example, even if the user understands the way data is visualized, the user may still be confused by the fact that important information is missing from the visualization, or the presented data is irrelevant.

As discussed above, adjusting data content selection 251 may comprise determining a data element of the data relevant to cognitive state 267 of the user based on gaze pattern 265 and adjusting the first data content setting 251 based on said data element. For example, additional information about the data element may be included in data 262 to be visualized. For example, shown in **Fig. 5c** is a visualization of a radiology report in which additional information 530 about the word "pancreatectomy", 531 is shown. For example, the user may have been looking at this word when or just before a cognitive state indicating confusion is detected. Various other types of additional information are possible, e.g., correlated or temporally related data. For example, a graph showing a time series hospital admissions per day may be augmented with information about a particular data point of the time series, e.g., weather or news about that day.

As another example, **Fig. 5d** shows an overall visualization of occupancy data of a hospital. Upon detecting that it is desirable to present the user with more information about the percentage shown in the figure, e.g., the user was puzzled while looking at the percentage of 85.9% shown in the figure, the visualization may be adapted by visualization selection unit 243 to show a more complex visualization with more information about the value, for example that in **Fig. 5e** showing floor-by-flow occupancy data.

Interestingly, in response to detecting a high confusion level, visualization selection unit 243 may be able both to present a simplified representation of the data, as exemplified by Fig. 5b, and a more complex visualization showing additional information, as exemplified by Fig. 5c and Fig. 5e. Namely, a simplified representation may be chosen in case of a global gaze pattern and a more complex visualization may be chosen in case of a local gaze pattern. Hence, the visualization can be controlled in a more efficient way that better matches the cognitive state of the user.

Returning now to **Fig. 2****,** in various embodiments, controlling system 200 may be configured to measure success of chosen data representation and/or data content settings, and/or to use previous experiences to determine the data representation and/or data content settings. In effect, an adaptive data visualization may be obtained in which visualizations are optimized by trying alternative graphical representation options and/or providing additional information, and observing if the user understanding improves. For example, system 200 can store which options work best and base the selection of data content settings 251, 261 and/or data representation settings 253, 263 based on such stored experiences. This way, the system is able to provide more suitable adjustments to settings 251, 253.

In an embodiment, cognition analysis unit 245 is configured to determine a cognitive state of the user for the second visualization of the data. Based on the cognitive state, visualization selection unit 243 may measure whether the choice of the second data content setting 261 and second data representation setting 262 was successful. For example, it may be determined that the user understands the data, e.g., has entered Phase 3, 440 as discussed with reference to Fig. 4. For example, determining that the user understands the data may be based on a relatively long gaze of the user away from the visualization, e.g., at least one second, and/or a gazing at a relatively small number of data elements. Detecting an increased understanding may indicate that the previous modification the visualization, e.g., to its content and/or representation, was successful. For example, visualization selection unit 243 may keep a list of modifications and associated success measures, e.g., changes in detected understanding by the user. The list of modifications may be stored with reference to a particular user and/or a particular type of visualization. The list of modifications may be for use by controlling system 200 and/or other controlling systems in determining data content and/or data representation settings. The list of modifications can also be used to support a learning system, e.g., to see what models work for what types of users and so help to define new evolving visualization standards.

In an embodiment, visualization selection unit 243 may determine data content settings 251, 261 and/or data representation settings 253, 263 based on previously measured success of chosen data content settings and data representation settings. For example, the previously measured success can be measured by device 200 itself, e.g., stored in the form of list of modifications above, and/or obtained from other devices, e.g., as a predefined or periodically updated list. For example, visualization selection unit 243 may select a modification with a highest average increase in understanding. Visualization selection unit 243 may also select a modification according to a model trained using training records comprising possible modifications to data representations settings and/or data content settings, e.g. labelled by a measured increase in understanding due to the respective settings. Such a trained model may be re-trained, e.g., periodically, based on new measurements. In some cases, non-optimal modifications, e.g., with lower expected increase in understanding, may be chosen, e.g., in an exploration-exploitation trade-off. As above, previously measured success only for the current user may be used, e.g., if enough such data is available, or previously measured success also for other users may be considered, e.g., to increase the amount of available data to base the modifications on.

In various embodiments, the second data representation setting 262 and second data content setting 261 are determined based on the user's gaze pattern 265 and/or cognitive state 267 without requiring further user interaction, e.g., the second visualization 264 can be shown without requiring any user selection or confirmation. This is particularly useful in settings where it is hard for the user to provide such as selection or confirmation, e.g., if the user is otherwise occupied, as already discussed.

In other embodiments, however, visualization selection unit 243 may adjust the first data representation setting 252 and/or first data content 251 setting by determining one or more visualization improvement suggestions and by determining said setting 251 or 252 based on one or more of said visualization improvement suggestions selected by the user. In such cases, the system may in effect serve as a digital assistant whose efficiency is improved by letting it provide more accurate suggestions.

Optionally, if a user shows confusion for a while, the digital assistant may ask questions to get a better understanding of what the user is looking for. Such questions can be scoped based on previous visualizations that the user has already seen.

Visualization selection unit 243 may determine one or more visualization improvement suggestions, for example, based on the user's answers, based on previously measured success as described above, etcetera. For example, the suggestions may include one or more of a different perspective on the data, different details to be highlighted, a comparison with other data, etc. The user may select such an improvement and second visualization 264 may be based on this selection.

If the user is still confused at the second visualization, the above process may be repeated, e.g., new suggestions may be presented to the user, e.g., based on questions from the digital assistant. If the user seems to experience insight, e.g., entering Phase 3 as described with respect to Fig. 4, then the system may also optionally ask about questions about this, e.g., to confirm the understanding, to provide further supporting info, to counteract false insights, etc. Thereby, a digital assistant is provided that allows to more efficiently control the data visualization, e.g., enabling the user to reach insights faster and/or reduce the amount of misunderstanding.

Controlling systems as described herein may be applied in various settings where it is desired to control a data inspection by a user. In particular, in various embodiments, the user may be a clinician. In a clinical environment, such users often need to deal with relatively complex data, in terms of quality and/or quantity. In particular, the data may comprise patient data from multiple modalities and/or data comparing the patient to a patient population according to multiple aspects. In the clinical setting, avoiding mistakes is particularly critical. Moreover, data inspection may need to happen under time pressure and/or as part of a task where the clinician is using his hands, etc. This makes the controlling systems as described herein particularly beneficial.

In various embodiments, controlling systems as described herein may be used to assist in a medical decision-making process for the clinician to provide a diagnosis and/or determine a course of treatment. An example of an environment in which such a medical decision-making process takes place is a tumor board meeting. In such a meeting, a clinician needs to make a medical decision in a short time, for example, in one or a few minutes. Data from various modalities may be present, e.g., medical images, e.g., MRI or CT scans; patient records; comparative data to other patients, etcetera. In various embodiments, the visualization comprises a clinical multidisciplinary dashboard, for example, comprising oncology data and/or cardiology data and/or ICU data and/or rehabilitation data. A visualization may for instance combine the multiple modalities in a dashboard presented to the user. The dashboard may be optimized in various ways based on the cognitive state and gaze pattern of the clinician, for example, by showing additional information related to particular data elements of the dashboard, and/or by presenting alternative visualizations for various data presented in the dashboard. Multiple clinicians may be present at decision-making meetings, although controlling systems as described herein are typically directed towards controlling a data inspection of a single clinician.

In various embodiments, controlling systems as described herein may be used to support a clinician, e.g., a cardiologist, in performing a medical procedure. For example, data being visualized may be from multiple modalities, e.g., the data may comprise live data about the patient, e.g., images, scans, vital sign readings, etcetera; data about the procedure taking place such as protocol or medical notes; comparative data of other patients, etc. In this case, since the clinician's mental focus is typically not entirely directed at the visualization and/or since the clinician is not able to use his hands for controlling the visualization, automatic adjustment of the visualization to optimize the cognitive understanding process of the clinician is particularly useful.

Controlling systems as described herein are useful for various other types of medical visualization. For example, a controlling system may be for controlling an inspection of population health data. Such data may compare a particular patient to a patient population, sometimes also called cohort, according to multiple aspects, e.g., based on age, illness history, physiological parameters, etc. Such visualizations are typically relatively complex since the multiple aspects are typically integrated into a single visualization, but may also give rise to surprising insights about the patient being inspected. Hence, being able to guide the user through the visualization by simplifying it or presenting additional insight depending on the needs of the patients, as achieved by various embodiments described herein, is useful.

Although medical data is used to illustrate the invention, the invention is by no means limited to it. For example, embodiments of the invention can be used to control an inspection of financial reporting data. This can be in the context of a hospital or other medical care institute, but can also be of another company or even of personal finances. Typically, financial reporting involves many different kinds of visualization and different kinds of data; moreover, since it may only take place once every few months, confusion about data representation is relatively likely. Other interesting application areas include social media dashboards, news dashboards, and analytics dashboards, e.g., for app and web data, company data, city planning data, traffic data, customer data, etcetera. Embodiments herein allow to control the visualization of such data with greater efficiency and less cognitive strain of the user.

Typically, the data inspection devices 110, 111, 210 each comprise a microprocessor which executes appropriate software stored at the respective devices; for example, that software may have been downloaded and/or stored in a corresponding memory, e.g., a volatile memory such as RAM or a non-volatile memory such as Flash. Alternatively, the devices may, in whole or in part, be implemented in programmable logic, e.g., as field-programmable gate array (FPGA). The devices may be implemented, in whole or in part, as a so-called application-specific integrated circuit (ASIC), e.g., an integrated circuit (IC) customized for their particular use. For example, the circuits may be implemented in CMOS, e.g., using a hardware description language such as Verilog, VHDL etc.

Execution of the data inspection devices presented herein may be implemented in a processor circuit, examples of which are shown herein. Figs. 2 and 4 show functional units that may be functional units of the processor circuit. For example, Fig. 2 may be used as a blueprint of a possible functional organization of the processor circuit. The processor circuit is not shown separate from the units in Fig. 2. For example, the functional units shown in Fig. 2 may be wholly or partially be implemented in computer instructions that are stored at device 210, e.g., in an electronic memory of device 210, and are executable by a microprocessor of device 210. In hybrid embodiments, functional units are implemented partially in hardware, e.g., as coprocessors, e.g., crypto coprocessors, and partially in software stored and executed on device 210. In an embodiment, the data inspection device comprises a data selection circuit, a data visualization circuit, a visualization selection circuit, a gaze analysis circuit, and/or a cognition analysis circuit. The device may comprise additional circuits. The circuits implement the corresponding units described herein. The circuits may be a processor circuit and storage circuit. A processor circuit may be implemented in a distributed fashion, e.g., as multiple sub-processor circuits.

In the various embodiments of data inspection devices 110, 111, 210, the communication interface may be selected from various alternatives. For example, the interface may be a network interface to a local or wide area network, e.g., the Internet, a storage interface to an internal or external data storage, a keyboard, an application interface (API), etc.

The data inspection device may have a user interface, which may include well-known elements such as one or more buttons, a keyboard, display, touch screen, etc. The user interface may be arranged for accommodating user interaction to control the data inspection.

Memory 131 may be implemented as an electronic memory, say a flash memory, or magnetic memory, say hard disk or the like. Memory 131 may be a storage. Memory 131 may also comprise multiple discrete memories together making up memory 131. Memory 131 may also be a temporary memory, say a RAM. In the case of a temporary storage 131, storage 131 contains some means to obtain data before use, say by obtaining them over an optional network connection.

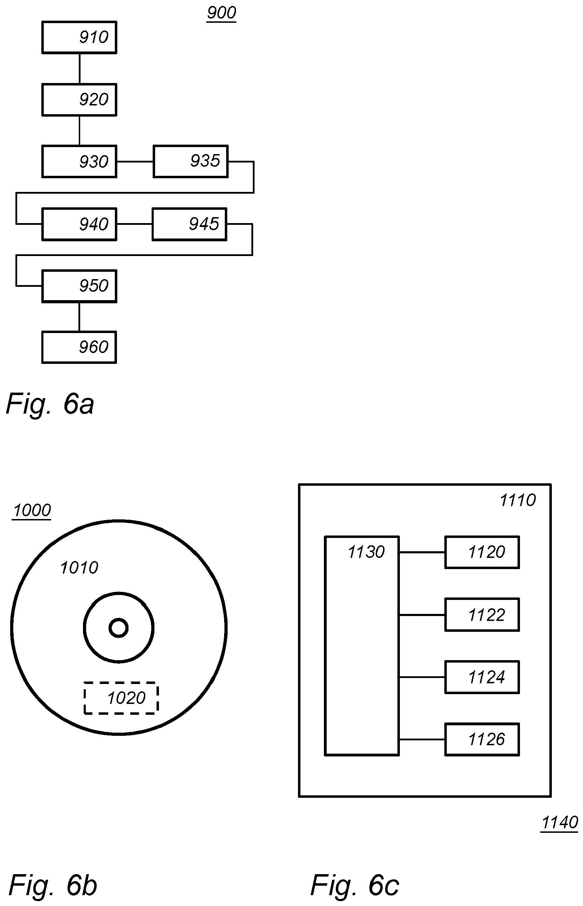

**Fig. 6a** schematically shows an example of a method 900 of controlling a data inspection by a user. Method 900 is typically computer-implemented. Method 900 may comprise storing 910 the data. Method 900 may comprise showing 920 a first visualization of the data. The first visualization may comprise a visualization according to a first data representation setting of a selection of the data according to a first data content setting. Method 900 may comprise sensing 930 data indicative of a gaze pattern of the user looking at the first visualization. Method 900 may further comprise determining 935 the gaze pattern of the user for said visualization based on said sensed data.

Method 900 may comprise sensing 940 data indicative of a cognitive state of the user looking at the first visualization. Method 900 may further comprise determining 945 the cognitive state of the user for said visualization based on said sensed data.

Method 900 may comprise determining 950, based on the gaze pattern and the cognitive state, a second data content setting and a second data representation setting.

Method 900 may comprise showing 960 a second visualization of the data. The second visualization may comprise a visualization according to the second data representation setting of a selection of the data according to the second data content setting.

Many different ways of executing the method are possible, as will be apparent to a person skilled in the art. For example, the order of the steps can be varied or some steps may be executed in parallel. Moreover, in between steps other method steps may be inserted. The inserted steps may represent refinements of the method such as described herein, or may be unrelated to the method. For example, steps 930 and 940 may be executed, at least partially, in parallel. Moreover, a given step may not have finished completely before a next step is started.

Embodiments of the method may be executed using software, which comprises instructions for causing a processor system to perform method 900. Software may only include those steps taken by a particular sub-entity of the system. The software may be stored in a suitable storage medium, such as a hard disk, a floppy, a memory, an optical disc, etc. The software may be sent as a signal along a wire, or wireless, or using a data network, e.g., the Internet. The software may be made available for download and/or for remote usage on a server. Embodiments of the method may be executed using a bitstream arranged to configure programmable logic, e.g., a field-programmable gate array (FPGA), to perform the method.

It will be appreciated that the invention also extends to computer programs, particularly computer programs on or in a carrier, adapted for putting the invention into practice. The program may be in the form of source code, object code, a code intermediate source, and object code such as partially compiled form, or in any other form suitable for use in the implementation of an embodiments of the method. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the means of at least one of the systems and/or products set forth.

**Fig. 6b** shows a computer readable medium 1000 having a writable part 1010 comprising a computer program 1020, the computer program 1020 comprising instructions for causing a processor system to perform a method of controlling a data inspection by a user, according to an embodiment. The computer program 1020 may be embodied on the computer readable medium 1000 as physical marks or by means of magnetization of the computer readable medium 1000. However, any other suitable embodiment is conceivable as well. Furthermore, it will be appreciated that, although the computer readable medium 1000 is shown here as an optical disc, the computer readable medium 1000 may be any suitable computer readable medium, such as a hard disk, solid state memory, flash memory, etc., and may be non-recordable or recordable. The computer program 1020 comprises instructions for causing a processor system to perform said method of controlling a data inspection by a user.

**Fig. 6c** shows in a schematic representation of a processor system 1140 according to an embodiment. The processor system comprises one or more integrated circuits 1110. The architecture of the one or more integrated circuits 1110 is schematically shown in Figure 7b. Circuit 1110 comprises a processing unit 1120, e.g., a CPU, for running computer program components to execute a method according to an embodiment and/or implement its modules or units. Circuit 1110 comprises a memory 1122 for storing programming code, data, etc. Part of memory 1122 may be read-only. Circuit 1110 may comprise a communication element 1126, e.g., an antenna, connectors or both, and the like. Circuit 1110 may comprise a dedicated integrated circuit 1124 for performing part or all of the processing defined in the method. Processor 1120, memory 1122, dedicated IC 1124 and communication element 1126 may be connected to each other via an interconnect 1130, say a bus. The processor system 1110 may be arranged for contact and/or contact-less communication, using an antenna and/or connectors, respectively.

For example, in an embodiment, processor system 1140, e.g., the data inspection device, may comprise a processor circuit and a memory circuit, the processor being arranged to execute software stored in the memory circuit. For example, the processor circuit may be an Intel Core i7 processor, ARM Cortex-R8, etc. In an embodiment, the processor circuit may be ARM Cortex M0. The memory circuit may be an ROM circuit, or a non-volatile memory, e.g., a flash memory. The memory circuit may be a volatile memory, e.g., an SRAM memory. In the latter case, the device may comprise a non-volatile software interface, e.g., a hard drive, a network interface, etc., arranged for providing the software.

It should be noted that the above-mentioned embodiments illustrate rather than limit the invention, and that those skilled in the art will be able to design many alternative embodiments.

In the claims, any reference signs placed between parentheses shall not be construed as limiting the claim. Use of the verb 'comprise' and its conjugations does not exclude the presence of elements or steps other than those stated in a claim. The article 'a' or 'an' preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

In the claims references in parentheses refer to reference signs in drawings of exemplifying embodiments or to formulas of embodiments, thus increasing the intelligibility of the claim. These references shall not be construed as limiting the claim.

## Claims

1. A system (101) for controlling a data inspection by a user, the system comprising:
- a memory (131) configured to store the data;
- a display (161) for showing a visualization of the data;
- a gaze pattern sensor (171) for sensing data indicative of a gaze pattern of the user looking at the visualization shown on the display;
- a cognitive state sensor (181) for sensing data indicative of a cognitive state of the user looking at the visualization shown on the display;
- a processor (141) configured to:
- show a first visualization of the data, comprising a visualization according to a first data representation setting of a selection of the data according to a first data content setting;
- determine the gaze pattern of the user for the first visualization of the data based on said data sensed by the gaze pattern sensor;
- determine the cognitive state of the user for the first visualization of the data based on said data sensed by the cognitive state sensor;
- determine, based on the gaze pattern and the cognitive state, a second data content setting and a second data representation setting;
- show a second visualization of the data, comprising a visualization according to the second data representation setting of a selection of the data according to the second data content setting.

2. The system (101) according to claim 1, wherein the user is a clinician, the data comprising patient data from multiple modalities and/or comparing the patient to a patient population according to multiple aspects.

3. The system (101) according to any one of the preceding claims, wherein determining the second data representation setting comprises determining a globality level of the gaze pattern and a confusion level of the cognitive state and adjusting the first data representation setting if said globality level is high and said confusion level is high.

4. The system (101) according to any one of the preceding claims, wherein determining the second data content setting comprises determining a globality level of the gaze pattern and a confusion level of the cognitive state and adjusting the first data content setting if said globality level is low and said confusion level is high.

5. The system (101) according to claim 4, wherein determining the second content setting comprises determining a data element of the data relevant to the cognitive state of the user based on the gaze pattern and adjusting the first data content setting based on said data element.

6. The system (101) according to any one of claims 3 to 5, wherein the gaze pattern comprises multiple points of the visualization on the display and corresponding gaze durations, determining the globality level of the gaze pattern comprising determining an average distance between points of the gaze pattern and/or an average gaze duration.

7. The system (101) according to any one of the preceding claims, wherein the gaze pattern sensor (171) comprises a camera for capturing image data of the gaze pattern of the user.

8. The system (101) according to any one of the preceding claims, wherein the cognitive state sensor (181) comprises one or more of:
- a camera for capturing image data of one or more of a facial expression of the user, a gaze pattern of the user, and a pupil dilation of the user;
- a heart rate sensor;
- a skin conductance sensor;
- a mental load sensor; and
- a voice tone sensor.

9. The system (101) according to any one of the preceding claims, wherein adjusting the first data representation setting and/or first data content setting comprises determining one or more visualization improvement suggestions and determining said setting based on one or more of said visualization improvement suggestions selected by the user.

10. The system (101) according to any one of the preceding claims, wherein the processor (141) is further configured to determine a cognitive state of the user for the second visualization of the data and to measure whether the choice of the second data content setting and second data representation setting was successful therefrom.

11. The system (101) according to claim 10, wherein determining whether said choice was successful comprises detecting a long gaze away from the visualization and/or long gazes at multiple points of the visualization.

12. The system (101) according to any one of the preceding claims, wherein the processor (141) is configured to determine one or more of the first data content setting, the second data content setting, the first data representation setting, and the second data representation setting based on previously measured success of chosen data content settings and data representation settings.

13. A data inspection device (111) for use in the system according to any one of the previous claims, comprising the memory (131) and a processor (141) configured to at least:
- show the first visualization of the data;
- determine, based on the determined gaze pattern and the determined cognitive state, the second data content setting and the second data representation setting;
- show the second visualization of the data.

14. A method (900) of controlling a data inspection by a user, the method comprising:
- storing (910) the data;
- showing (920) a first visualization of the data, comprising a visualization according to a first data representation setting of a selection of the data according to a first data content setting;
- sensing (930) data indicative of a gaze pattern of the user looking at the first visualization and determining (935) the gaze pattern of the user for said visualization based on said sensed data;
- sensing (940) data indicative of a cognitive state of the user looking at the first visualization and determining (945) the cognitive state of the user for said visualization based on said sensed data;
- determining (950), based on the gaze pattern and the cognitive state, a second data content setting and a second data representation setting;
- showing (960) a second visualization of the data, comprising a visualization according to the second data representation setting of a selection of the data according to the second data content setting.

15. A computer readable storage medium (1000) comprising transitory or non-transitory data (1020) representing instructions to cause a processor system to perform the method according to claim 14.
